# EUROPEAN PATENT SPECIFICATION

(11) **EP 3 750 487 B1**
(45) Date of publication and mention of the grant of the patent: **04.11.2026**
(21) Application number: 19750339.4
(22) Date of filing: 08.02.2019
(51) Int. Cl.: A61B 17/11, A61B 17/072, A61F 2/04, A61L 31/06, A61L 31/14, A61F 2/00, A61B 17/00, A61B 17/115, A61B 17/04

(54) **ADHESION PROMOTING DEVICE**
HAFTFÖRDERUNGSVORRICHTUNG
DISPOSITIF FAVORISANT L'ADHÉRENCE

(30) Priority: 08.02.2018 JP 2018021171
(43) Date of publication of application: 16.12.2020
(73) Proprietor: Terumo Kabushiki Kaisha, Tokyo 151-0072 (JP)
(72) Inventor: HATA, Mayu, Ashigarakami-gun, Kanagawa 259-0151 (JP); KAI, Miho, Ashigarakami-gun, Kanagawa 259-0151 (JP); ARAMAKI, Naoki, Ashigarakami-gun, Kanagawa 259-0151 (JP)
(74) Representative: Casalonga
(86) International application number: PCT/JP2019/004690
(87) International publication number: WO 2019/156230

(56) References cited:
- WO-A2-2010/052584
- JP-A- 2006 110 356
- JP-A- 2007 229 457
- JP-A- 2007 505 708
- JP-A- 2011 015 966
- JP-A- 2017 521 134
- JP-A- H01 274 750
- US-A1- 2005 059 996
- US-A1- 2006 108 393
- US-A1- 2010 012 703
- US-A1- 2011 262 696
- US-A1- 2011 288 568
- US-A1- 2011 288 568
- US-A1- 2013 204 078

## Description

### Technical Field

The present invention relates to an adhesion promoting device.

### Background Art

The surgical indication of a surgery is determined in each individual by comparing a benefit derived from the surgery with a surgical wound (a wound such as an incisional wound and an anastomotic wound). That **is,** the surgery is performed in a case where the benefit of the surgery surpasses the disbenefit of surgical invasion (surgical indication). Therefore, in a case where the benefit derived from the surgery is large, but a living body is not likely to withstand the surgical invasion and to recover, the surgery is not performed (contraindication). However, in practice, there are many cases where it is difficult to determine a balance between the disbenefit of the surgery due to a damage and the benefit of intention, and there are many cases where it is not allowed to be optimistic even in a case where the surgery appears to be beneficial before the surgery (with surgery indication). This is caused by many factors existing before the surgery, during the surgery, and after the surgery, and is affected by a factor such as individual reserve power, cardiopulmonary function, and infection, and thus, an unexpected result of the surgery may be obtained. For example, on an anatomical form, in the surgery of the digestive canal (subtotal esophagectomy with respect to esophagus cancer, pancreatoduodenectomy with respect to pancreatic cancer, colectomy with respect to colorectal cancer, and the like) in which circulation disorder is easily caused and there is no means for improving circulation, when a ruptured suture occurs in a suture site, contents are leaked from a ruptured anastomosis site, and thus, inflammation may be caused. Then, an inflammation symptom such as fever is caused, and thus, in a critical case, fatal complication such as peritoneum inflammation or ichorrhemia may occur, and abrosia or antibacterial agent delivery, and a reoperative surgery may be required.

As described above, in particular, in a surgery for the digestive canal, the most important prognosis determination factor is that wound healing (adhesion) in the anastomosis site is not delayed.

As means for preventing such a ruptured suture, for example, in colorectal cancer that is known as having a high occurrence rate of the ruptured suture (in particular, lower advanced rectal cancer), in order to reduce the influence of a surgery factor in the causes of the ruptured suture, an anastomotic method in which a blood flow is maintained, and an excessive tension is not applied has been considered. In addition, many proposals for reducing the risk of the ruptured suture by performing stapled anastomosis using an anastomotic appliance such as a circular stapler have been made (for example, refer to JP-T-2007-505708 (the term "JP-T" as used herein means a published Japanese translation of a PCT patent application) (WO 2005/027983 A)). Other perforated medical devices are known from US 2011/288568, WO 2010/052584 and US 2010/012703. More particularly, US 2011/288568 describes an implantable prosthesis to cover the tissue or muscle wall defect. It includes a perforated sheet of a biologically compatible material. WO 2010/052584 relates to a medical device comprising a perforated bacterial cellulose sheet and used when soft tissues need to be repaired, reinforced or replaced. US 2010/012703 discloses an annular gasket for use with a circular stapling apparatus in performing an anastomosis. The annular gasket comprises a plurality of pores extending through its body portion.

### Summary of Invention

### Technical Problem

However, even in a case of using the stapled anastomosis as described in JP-T-2007-505708 (WO 2005/027983 A), a progress degree of the wound healing (the adhesion) in the anastomosis site depends on a patient factor such as a tissue state of a patient, and thus, the risk of the ruptured suture still remains. In addition, it is necessary for an operator to be skilled in the manipulation of a dedicated anastomotic appliance in order to perform the stapled anastomosis, and thus, it is difficult to say that such a method is a simple solution.

Therefore, an object of the invention is to provide means for reducing the risk of a ruptured suture after a surgery or the like by a simple technique.

### Solution to Problem

The inventors have conducted intensive studies for attain the object described above. As a result thereof, the inventors have found that wound healing (adhesion) in an anastomosis site at a surgery or the like is promoted by using a biodegradable sheet having a plurality of through-holes of which the profile of a hole diameter and a pitch is controlled, and thus, have completed the invention.

That is, according to the invention, a medical apparatus including a biodegradable sheet in which a plurality of through-holes are formed is provided. Here, a value of a ratio (D/P) of a hole diameter D to a pitch P of the through-hole is controlled in a range of 1.5 to 20. The medical apparatus is used as an adhesion promoting device that is used for promoting the adhesion of a biological tissue.

### Brief Description of Drawings

[Fig. 1] Fig. 1 is an overview perspective view of a biodegradable sheet configuring a medical apparatus according to one embodiment of the invention.
[Fig. 2] Fig. 2 is an enlarged view of a part of a sectional surface along line 2-2 illustrated in Fig. 1.
[Fig. 3] Fig. 3 is an enlarged view for describing the size of a plurality of through-holes, and is a plan view seen from a direction of an arrow 3 in Fig. 1.
[Fig. 4] Fig. 4 is an overview perspective view of a biodegradable sheet according to Modification Example 1 of the embodiment.
[Fig. 5] Fig. 5 is an enlarged view of a part of a sectional surface of a biodegradable sheet according to Modification Example 2 of the embodiment.
[Fig. 6] Fig. 6 is an enlarged view of a part of a sectional surface of a biodegradable sheet according to Modification Example 3 of the embodiment.
[Fig. 7] Fig. 7 is an overview perspective view of a biodegradable sheet according to Modification Example 4 of the embodiment.
[Fig. 8] Fig. 8 is an overview perspective view of a biodegradable sheet according to Modification Example 5 of the embodiment.
[Fig. 9] Fig. 9 is an overview perspective view of a biodegradable sheet according to Modification Example 6 of the embodiment.
[Fig. 10] Fig. 10 is an overview perspective view of a biodegradable sheet according to Modification Example 7 of the embodiment.
[Fig. 11] Fig. 11 is an overview perspective view of a biodegradable sheet according to Modification Example 8 of the embodiment.

### Description of Embodiments

Hereinafter, an embodiment of the invention will be described with reference to the attached drawings.

### <<Medical Apparatus (Biodegradable Sheet)>>

One aspect of the disclosure relates to a medical apparatus including a biodegradable sheet in which a plurality of through-holes are formed, in which a value of a ratio (D/P) of a hole diameter D to a pitch P of the through-hole is 0.25 or more and less than 40. According to the present invention, the value of the ratio (D/P) of the hole diameter D to the pitch P of the through-holes is 1.5 to 20. According to the medical apparatus according to this aspect, it is possible to reduce the risk of a ruptured suture after a surgery or the like by a simple technique of interposing the medical apparatus between anastomosis sites (suture sites). Hereinafter, this aspect will be described in detail.

The medical apparatus according to this aspect includes the biodegradable sheet.

### <Constituent Material of Biodegradable Sheet>

A constituent material of the biodegradable sheet is not particularly limited, and examples of the constituent material include a biodegradable resin. For example, a known biodegradable (co)polymer described in JP-T-2011-528275, JP-T-2008-514719, WO 2008-1952 A, JP-T-2004-509205, and the like can be used as the biodegradable resin. Specifically, examples of the biodegradable resin include (1) a polymer selected from the group consisting of aliphatic polyester, polyester, polyacid anhydride, polyorthoester, polycarbonate, polyphosphazene, polyphosphoric acid ester, polyvinyl alcohol, polypeptide, polysaccharide, protein, and cellulose; (2) a copolymer containing one or more monomers configuring (1) described above, and the like. That is, it is preferable that the biodegradable sheet contains at least one type of biodegradable resin selected from the group consisting of the polymer selected from the group consisting of the aliphatic polyester, the polyester, the polyacid anhydride, the polyorthoester, the polycarbonate, the polyphosphazene, the polyphosphoric acid ester, the polyvinyl alcohol, the polypeptide, the polysaccharide, the protein, and the cellulose, and the copolymer containing one or more monomers configuring the polymer.

Here, the aliphatic polyester is not particularly limited, and examples of the aliphatic polyester include a polylactic acid (PLA) such as a poly-L-lactic acid, a poly-D-lactic acid, and a poly-DL-lactic acid, a polyglycolic acid (PGA), a polyhydroxybutyric acid, a polyhydroxyvaleric acid, a polyhydroxypentanoic acid, a polyhydroxyhexanoic acid, a polyhydroxyheptanoic acid, polycaprolactone, polytrimethylene carbonate, polydioxanone, a polymalic acid, polyethylene adipate, polyethylene succinate, polybutylene adipate, polybutylene succinate, and the like. In addition, the polycarbonate is not particularly limited, and examples of the polycarbonate include tyrosine-derived polycarbonate and the like.

Alternatively, the biodegradable resin configuring the biodegradable sheet may contain a copolymer in which monomers configuring a polymer are arbitrarily copolymerized. Here, the copolymer is not particularly limited. Specifically, examples of the copolymer include a lactic acid-caprolactone copolymer, a caprolactone-glycolic acid copolymer, PLGA (poly(lactide-co-glycolide), polyanhydride, polyorthoester, poly(N-(2-hydroxypropyl) methacrylamide), DLPLA-poly(dl-lactide), LPLA-poly(l-lactide), PGA-polyglycolide, PDO-poly(dioxanone), PGA-TMC-poly(glycolide-co-trimethylene carbonate), PGA-LPLA-poly(l-lactide-co-glycolide), PGA-DLPLA-poly(dl-lactide-co-glycolide), LPLA-DLPLA-poly(l-lactide-co-dl-lactide), and PDO-PGA-TMC-poly(glycolide-co-trimethylene carbonate-co-dioxanone)), and polyanhydrideesters (PAE)-salicylate in which a salicylic acid is chemically introduced into a polymer main chain (for example, a polymer in which a salicylic acid is bonded to both terminals of a polylactide anhydride or a polyadipic acid), and the like.

The polymers and the copolymers described above may be respectively independently used, two or more types thereof may be used by being combined, or one or more types of polymers and one or more types of copolymers may be used by being combined. In addition, the polymers and the copolymers described above may be respectively produced by synthesis or commercially available products may be used. A synthesis method is not particularly limited, and a known method can be similarly applied or can be applied by being suitably modified. For example, the polylactic acid (PLA), the polyglycolic acid (PGA), or the lactic acid-glycolic acid copolymer (PLGA) can be obtained by selecting a material having a required structure from an L-lactic acid, a D-lactic acid, and a glycolic acid, as a raw material, and by performing dehydration polycondensation. Preferably, the polylactic acid (PLA), the polyglycolic acid (PGA), or the lactic acid-glycolic acid copolymer (PLGA) can be obtained by selecting a material having a required structure from lactide that is a cyclic dimer of a lactic acid and glycolide that is a cyclic dimer of a glycolic acid, and by performing ring-opening polymerization. The lactide includes L-lactide that is a cyclic dimer of an L-lactic acid, D-lactide that is a cyclic dimer of a D-lactic acid, meso-lactide in which a D-lactic acid and an L-lactic acid are subjected to cyclic dimerization, and DL-lactide that is a racemic mixture of the D-lactide and the L-lactide. In the invention, any lactide can be used.

A weight average molecular weight of the biodegradable resin is not particularly limited insofar as a moderate biodegradation rate can be exhibited. Specifically, it is preferable that the weight average molecular weight of the biodegradable resin is 10,000 or more. That is, it is preferable that the biodegradable sheet contains a biodegradable resin having a weight average molecular weight of 5,000 or more. The weight average molecular weight of the biodegradable resin according to the invention is more preferably 8,000 to 200,000, and is even more preferably 10,000 to 150,000. Note that, a measurement method of the weight average molecular weight is a gel permeation chromatography (GPC).

In the biodegradable resins described above, the polylactic acid (PLA), the polyglycolic acid (PGA), the polycaprolactone, the lactic acid-caprolactone copolymer, the caprolactone-glycolic acid copolymer, the lactic acid-glycolic acid copolymer (PLGA), the tyrosine-derived polycarbonate (tyrosine-polycarbonate), the polyanhydride esters (PAE) -salicylate are preferable, and the PLA, the PGA, or the PLGA is particularly preferable. This is because such biodegradable resins have high biocompatibility, and easily control decomposition in a living body. Note that, a copolymerization ratio of PGA/PLA in the PLGA is not particularly limited, but it is preferable that PGA/PLA is 50/50 or more and less than 100/0, in a molar ratio.

### <Shape of Biodegradable Sheet>

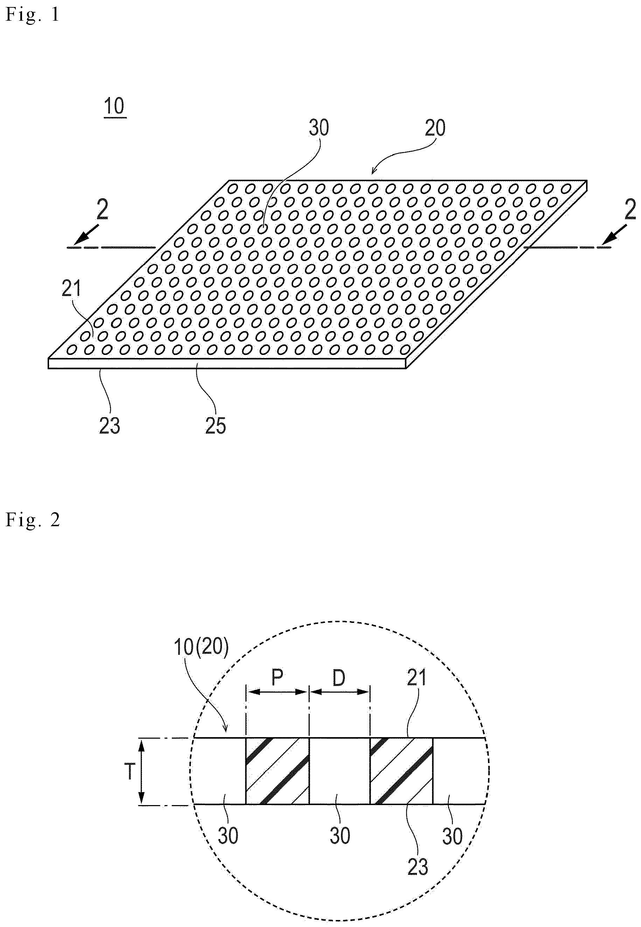

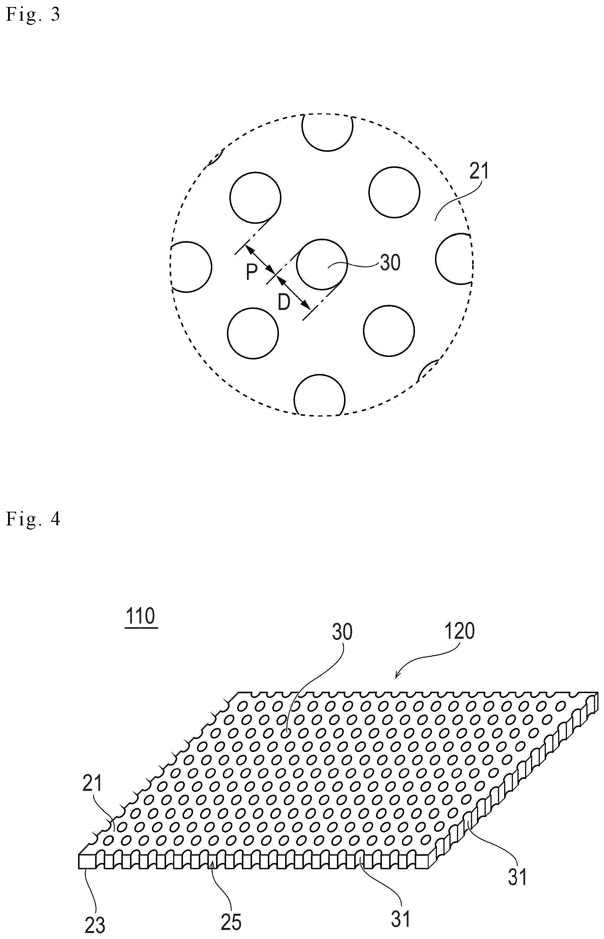

Fig. 1 is a plan view of the biodegradable sheet configuring the medical apparatus according to one embodiment of the invention. Fig. 2 is a sectional view along line 2-2 illustrated in Fig. 1. Fig. 3 is an enlarged explanatory diagram for describing the size of the plurality of through-holes.

As illustrated in Fig. 1, a biodegradable sheet 20 configuring a medical apparatus 10 is in the shape of a sheet. A plurality of through-holes 30 are formed in the biodegradable sheet 20. In Fig. 1, the plurality of through-holes 30 are regularly and periodically provided. The plurality of through-holes 30 may be randomly provided, and it is preferable that the plurality of through-holes are regularly and periodically provided. In addition, in Fig. 1, the plurality of through-holes 30 are provided perpendicularly along a thickness direction of the biodegradable sheet 20 (an up-down direction of Fig. 2). The plurality of through-holes 30 may be provided to be curved from an opening portion facing one surface (a front surface) 21 of the biodegradable sheet 20 towards an opening portion facing the other surface (a back surface) 23, and it is preferable that the plurality of through-holes are provided approximately perpendicularly along the thickness direction of the biodegradable sheet 20, and it is preferable that the plurality of through-holes are provided perpendicularly along the thickness direction of the biodegradable sheet 20.

The thickness of the biodegradable sheet 20 (a size T illustrated in Fig. 2) is not particularly limited, but is preferably 0.05 mm to 0.3 mm. According to the present invention, the thickness of the biodegradable sheet is 0.1 mm to 0.2 mm. In a case where the thickness of the biodegradable sheet 20 is 0.05 mm or more (in particular, 0.1 mm or more), it is possible to ensure a sufficient strength to the extent that the biodegradable sheet is not broken at handling. On the other hand, in a case where the thickness of the biodegradable sheet 20 is 0.3 mm or less (in particular, 0.2 mm or less), it is possible to ensure sufficient flexibility for the biodegradable sheet to closely follow a tissue to which the biodegradable sheet is applied.

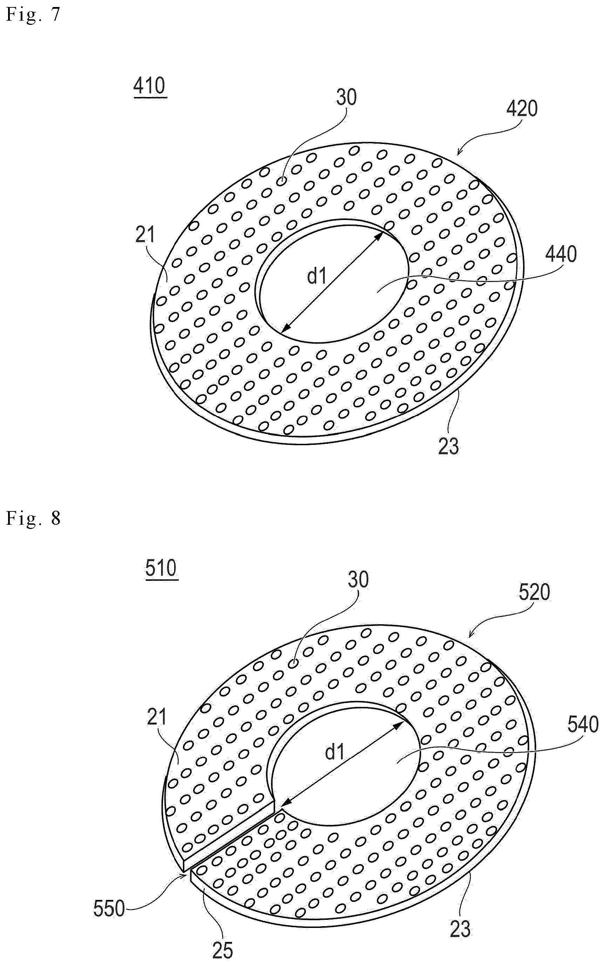

As illustrated in Fig. 1, the biodegradable sheet 20 according to this embodiment has an approximately rectangular shape, in a plan view. However, the outer shape of the biodegradable sheet 20 is not particularly limited, and for example, may be a circular shape (refer to Fig. 7), an elliptical shape, or the like.

The biodegradable sheet 20 configuring the medical apparatus according to the invention has a characteristic in the profile of the size of the plurality of through-holes 30. Specifically, the value of the ratio of the hole diameter D (a distance D illustrated in Fig. 3) of the plurality of through-holes 30 to the pitch P (a distance P illustrated in Fig. 3) of the plurality of through-holes 30 is 0.25 or more and less than 40. According to the present invention, the value of the ratio (D/P) of the hole diameter D to the pitch P of the through-holes is 1.5 to 20.

Here, in a case where the through-hole 30 is in the shape of a true circle, in a plan view, the hole diameter D of the through-hole 30 is identical to the diameter of the true circle. On the other hand, in a case where the through-hole 30 is not in the shape of a true circle, in a plan view, the diameter of the true circle (an equivalent circle diameter) having the same area as the area of the opening portion of the through-hole 30 is set to the hole diameter D of the through-hole 30. In the invention, the biodegradable sheet 20 includes the plurality of through-holes 30, and thus, there are a plurality of values of the hole diameters D respectively corresponding to the through-holes 30. Therefore, in the calculation of the value of the ratio described above, an arithmetic average value of two or more of the values of the hole diameters D respectively corresponding to the plurality of through-holes 30 is used as a representative value of the hole diameter D. On the other hand, the "pitch P" of the plurality of through-holes 30 indicates the shortest distance between the opening portions of two through-holes 30. In the value of the pitch P, there are a plurality of values of the pitches P respectively corresponding to combinations of the adjacent through-holes 30. Therefore, in the calculation of the value of the ratio described above, an arithmetic average value of two or more of the values of the pitches P respectively corresponding to the combinations of the adjacent through-holes 30 is used as a representative value.

As described above, it is essential that the value of the ratio (Hole Diameter/Pitch Ratio (D/P)) of the hole diameter D of the plurality of through-holes 30 to the pitch P of the plurality of through-holes 30 is 0.25 or more and less than 40. The value of the ratio (D/P) is preferably 0.5 to 35, is more preferably 1.0 to 30. According to the present invention, the value of the ratio (D/P) is 1.5 to 20. Here, as a mechanism in which adhesion is promoted by the medical apparatus 10 (the biodegradable sheet 20) according to the invention, it is assumed that the constituent material configuring the biodegradable sheet 20, such as the biodegradable resin, causes a biological reaction, and thus, the expression of a biological component such as fibrin is induced, and the biological component that is induced as described above is accumulated through the through-hole of the biodegradable sheet to achieve the adhesion. Therefore, in a case where the value of the Hole Diameter/Pitch ratio (D/P) described above is excessively small (less than 0.25), the hole diameter is excessively relatively small with respect to the pitch, and thus, even in a case where the fibrin or the like is induced, the accumulation through both surfaces of the sheet does not sufficiently occur, and it is not possible to promote the adhesion. On the other hand, in a case where the value of the Hole Diameter/Pitch ratio (D/P) described above is excessively large (40 or more), the pitch is excessively relatively small with respect to the hole diameter, and thus, it is not possible to sufficiently induce the biological component such as the fibrin, and it is not also possible to promote the adhesion.

Note that, a specific value of each of the hole diameter D and the pitch P is not particularly limited, but the hole diameter D is 0.1 mm to 6 mm, is preferably 0.3 mm to 4 mm, and is more preferably 0.6 mm to 1.5 mm. In addition, the pitch P is 0.1 mm to 0.4 mm, and is preferably 0.2 mm to 0.4 mm.

### <Blend of Adhesion Enhancement Agent>

An adhesion enhancement agent for promoting the adhesion may be blended in the biodegradable sheet configuring the medical apparatus according to the invention. The adhesion enhancement agent is not particularly limited insofar as a material has a synechia function in pleurodesis. Examples of the adhesion enhancement agent include talc, povidone-iodine (Isodine or the like), a curing agent (minocycline, tetracycline, or the like), a carcinostatic agent (adriamycin, mitomycin C, cisplatin, or the like), and an immunomodulator (OK-432, Broncasma Berna or the like). In a case where the talc, the povidone-iodine, the curing agent, and the carcinostatic agent are delivered into a pleural cavity, inflammation is caused by a chemical function, and in a recovery process, synechia occurs. In addition, the immunomodulator promotes fibrin deposition from the pleura by an allergic reaction with respect to killed bacterium preparation, and thus, promotes the synechia.

### <Manufacturing Method of Biodegradable Sheet>

A manufacturing method of a biodegradable sheet according to the invention is not particularly limited, and an arbitrary method capable of manufacturing a sheet containing the biodegradable resin or the like in which the through-holes having the characteristic size described above are formed can be adopted.

An example of the manufacturing method of the biodegradable sheet 20 includes a method of preparing a fiber containing the biodegradable resin described above, and of manufacturing a sheet in the shape of a mesh by using the fiber. The method of preparing the fiber containing the biodegradable resin is not particularly limited, and examples of the method include an electrospinning method (an electric field spinning method and an electrostatic spinning method), a melt-blowing method, and the like. Note that, in the invention, only one type of the methods may be selected and used, or two or more types thereof may be selected and suitably combined. Here, the electrospinning method is a method of forming a microfiber containing a resin, in a state where a high voltage (for example, approximately 20 kV) is applied between a syringe filled with a solution of the resin and a collector electrode. In a case of adopting such a method, the solution extruded from the syringe is electrically charged and is scattered in an electric field, but a solvent contained in the scattered solution is evaporated over time, and thus, a thinned solute appears. The thinned solute becomes the microfiber containing the resin, and is attached to a collector such as a substrate.

A mesh-like base material containing stainless (SUS) steel is used as the collector of the electrospinning method, and thus, a microfiber containing the biodegradable resin as the thinned solute is attached to a real portion of the mesh, and therefore, a mesh containing the microfiber is formed. A resin mesh obtained as described above is peeled off from the mesh-like base material, and thus, it is possible to manufacture the biodegradable sheet according to the invention. Note that, it is possible to control the shape of the biodegradable sheet (the hole diameter and the pitch of the through-hole) containing the resin mesh to be manufactured, by suitably adjusting the size of the mesh-like base material (the hole diameter and the pitch).

In addition, similarly, a method of forming a through-hole after obtaining a resin sheet having a homogeneous thickness by scattering the solution described above on the surface of a flat base material that is not in the shape of a mesh such that the microfiber is attached thereto can also be adopted as another example of the manufacturing method using the electrospinning method. In this case, for example, it is possible to form the through-hole in an irradiation site by irradiating the resin sheet with laser light condensed by using a condenser lens. Then, it is possible to control the shape of the biodegradable sheet (the hole diameter and the pitch of the through-hole) containing the resin mesh to be manufactured, by adjusting the energy or an irradiation time of the laser light to be emitted, the interval of the irradiation sites, and the like.

Note that, as still another example of the manufacturing method of the biodegradable sheet, the biodegradable sheet according to the invention may be manufactured by spinning the fiber containing the biodegradable resin described above in accordance with an ordinary method, and by knitting a fiber that is obtained into the shape of a mesh.

### <<Application of Medical Apparatus (Biodegradable Sheet)>>

The medical apparatus (the biodegradable sheet) according to the invention can be used as an adhesion promoting device that is used for promoting the adhesion of the biological tissue.

A specific type of disease (surgery) to which the medical apparatus (the biodegradable sheet) according to the invention can be applied as the adhesion promoting device is not particularly limited, and the medical apparatus (the biodegradable sheet) can also be applied to any disease (surgery) in which it is preferable for a living body to promote the adhesion through the biodegradable sheet. Among them, on an anatomical form, in the surgery of the digestive canal (subtotal esophagectomy with respect to esophagus cancer, pancreatoduodenectomy with respect to pancreatic cancer, colectomy with respect to colorectal cancer, and the like) in which circulation disorder is easily caused and there is no means for improving circulation, in order to prevent the ruptured suture in the anastomosis site, it is preferable to apply the medical apparatus (the biodegradable sheet) according to the invention to the anastomosis site in the surgeries, as the adhesion promoting device.

Hereinafter, each modification example of the embodiment described above will be described. Note that, in the description of each of the modification examples, the configuration and the like that are not particularly mentioned can be the same as those in the embodiment described above, and the description thereof will be suitably omitted.

### <Modification Example 1>

Fig. 4 illustrates a medical apparatus 110 (a biodegradable sheet 120) according to Modification Example 1.

In the medical apparatus 110 according to Modification Example 1, communicating holes 31 that are exposed to a lateral surface 25 of the biodegradable sheet 120 are formed. The communicating holes 31, for example, may be or may not be communicated with the through-holes 30 penetrating through one surface 21 and the other surface 23 of the biodegradable sheet 120. In addition, in a case where the biodegradable sheet 120 has an approximately rectangular outer shape, the communicating holes 31 may be formed in only one lateral surface 25 in four lateral surfaces 25, or the communicating holes 31 may be formed in a plurality of arbitrary lateral surfaces 25 in four lateral surfaces 25. Note that, a specific shape (the shape in a front view), the size, and the like of the communicating hole 31 are not particularly limited.

As with the biodegradable sheet 120 according to this modification example, in a case where the communicating holes 31 are formed in the lateral surface 25, a contact area with a living body increases, and thus, more robust adhesion can be attained.

### <Modification Example 2>

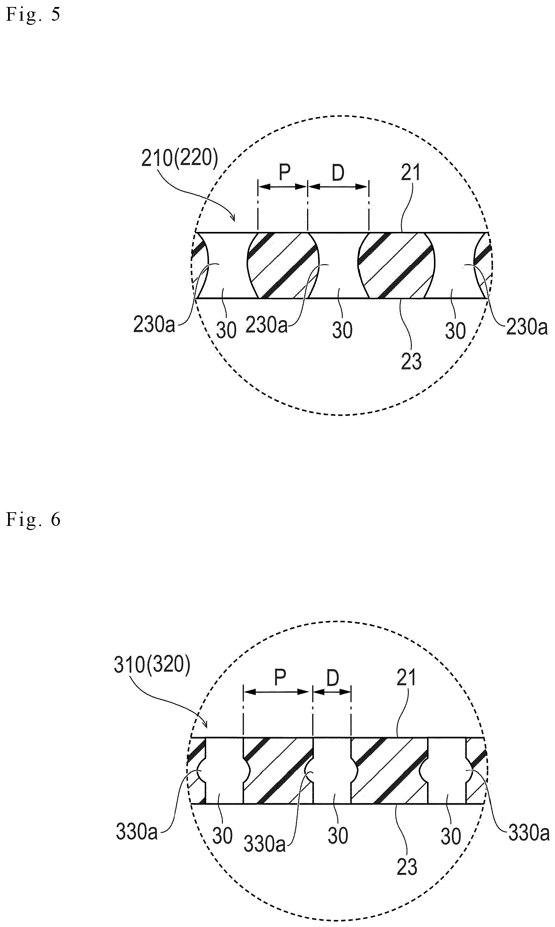

Fig. 5 illustrates an enlarged sectional view (a sectional view corresponding to Fig. 2) of a medical apparatus 210 (a biodegradable sheet 220) according to Modification Example 2.

As illustrated in Fig. 5, the through-holes 30 formed in the biodegradable sheet 220 have a shape in which a part widens in a direction intersecting with a thickness direction of the biodegradable sheet 220 (an up-down direction of Fig. 5). Specifically, as illustrated in Fig. 5, the through-holes 30 have a sectional shape in which the diameter is gradually narrowed from both of one surface 21 and the other surface 23 towards the approximately central position in the thickness direction. Accordingly, a narrow portion 230a that is formed to have a diameter smaller than other portions in the vicinity of the approximately central position in the thickness direction of the biodegradable sheet 220 is formed in the through-hole 30.

Note that, the Hole Diameter/Pitch ratio (D/P) of the biodegradable sheet 220 according to this modification example can also be calculated by the same technique as described above.

As with the biodegradable sheet 220 according to this modification example, in a case where the through-hole 30 is formed to include the narrow portion 230a, it is possible to reliably fill the through-holes with a smaller amount of biological component.

### <Modification Example 3>

Fig. 6 illustrates an enlarged sectional view (a sectional view corresponding to Fig. 2) of a medical apparatus 310 (a biodegradable sheet 320) according to Modification Example 3.

As illustrated in Fig. 6, the through-holes 30 have a shape in which a part widens in a direction intersecting with a thickness direction of the biodegradable sheet 320 (an up-down direction of Fig. 6). Specifically, as illustrated in Fig. 6, in the approximately central position in a thickness direction of the through-hole 30, a part widens in the direction intersecting with the thickness direction. Accordingly, a wide portion 330a that is formed to include a diameter larger than other portions in the approximately central position in the thickness direction of the biodegradable sheet 220 is formed in the through-hole 30.

Note that, the Hole Diameter/Pitch ratio (D/P) of the biodegradable sheet 320 according to this modification example can also be calculated by the same technique as described above.

As with the medical apparatus 310 according to this modification example, in a case where the through-hole 30 is formed to include the wide portion 330a, it is possible to accumulate a larger amount of biological components, and thus, it is possible to attain more robust adhesion.

### <Modification Example 4>

Fig. 7 illustrates an overview perspective view of a medical apparatus 410 (a biodegradable sheet 420) according to Modification Example 4.

As illustrated in Fig. 7, the biodegradable sheet 420 has a circular shape in a plan view. In addition, the biodegradable sheet 420 includes a central hole 440 that is formed to have a hole diameter d1 larger than that of the through-hole 30, in the approximately central position in the plan view.

The central hole 440, for example, can be formed to have a hole diameter of 5 mm to 25 mm. In addition, the outer shape of the central hole 440, for example, can be a true-circular shape, and may be an elliptical shape or a rectangular shape, other shapes, or the like.

As with the medical apparatus 410 according to this modification example, in a case where the biodegradable sheet 420 has a circular shape in a plan view, and includes the central hole 440 that is formed to have the hole diameter d1 larger than that of the through-hole 30, in the approximately central position in the plan view, it is possible to preferably use the biodegradable sheet 420 in a predetermined manipulation (for example, colorectal anastomosis using an automatic anastomosis device provided with a trocar and an anvil). In a case of using the biodegradable sheet 420 in the manipulation described above, for example, the biodegradable sheet 420 is set such that the central hole 440 is inserted into the trocar of the automatic anastomosis device. Then, the trocar and the anvil are engaged with each other in a state where the biodegradable sheet 420 is set in the trocar, and thus, it is possible to dispose the biodegradable sheet 420 between the intestinal walls of the intestinal canal that is an anastomosis target.

### <Modification Example 5>

Fig. 8 illustrates an overview perspective view of a medical apparatus 510 (a biodegradable sheet 520) according to Modification Example 5.

As illustrated in Fig. 8, the biodegradable sheet 520 has a circular shape in a plan view. In addition, the biodegradable sheet 520 includes a central hole 540 that is formed to have the hole diameter d1 larger than that of the through-hole 30, in the approximately central position in the plan view. In addition, the biodegradable sheet 520 includes a slit 550 continuing to the lateral surface 25 of the biodegradable sheet 520 and the central hole 540.

The central hole 540, for example, can be formed to have a hole diameter of 1 mm to 20 mm. In addition, the slit 550, for example, can be formed in a linear shape, and may be in a shape in which curves and straight lines meander (a zigzag shape), or the like, in a plan view.

As with the medical apparatus 510 according to this modification example, in a case where the biodegradable sheet 520 has a circular shape in a plan view, and includes the central hole 540 that is formed to have the hole diameter d1 larger than that of the through-hole 30, in the approximately central position in the plan view, and the slit 550 is formed, in a predetermined manipulation (pancreatic parenchyma-jejunum seromuscular anastomosis), it is possible to preferably use the biodegradable sheet 520. In a case of using the biodegradable sheet 520 in the manipulation described above, for example, the biodegradable sheet 520 is retained on the pancreatic parenchyma side by allowing the ductus pancreaticus exposed from the pancreatic parenchyma to pass through the slit 550. In a state where the biodegradable sheet 520 is retained as described above, an anastomotic surface (a cutoff surface) of the pancreatic parenchyma is moved close to the jejunum (the jejunum with a small hole into which the ductus pancreaticus is inserted) that is an anastomosis target, and thus, it is possible to dispose the biodegradable sheet 520 between the jejunum and the pancreatic parenchyma. The jejunum and the pancreatic parenchyma are sutured in a state where the biodegradable sheet 520 is disposed therebetween, and thus, it is possible to perform the anastomosis.

### <Modification Example 6>

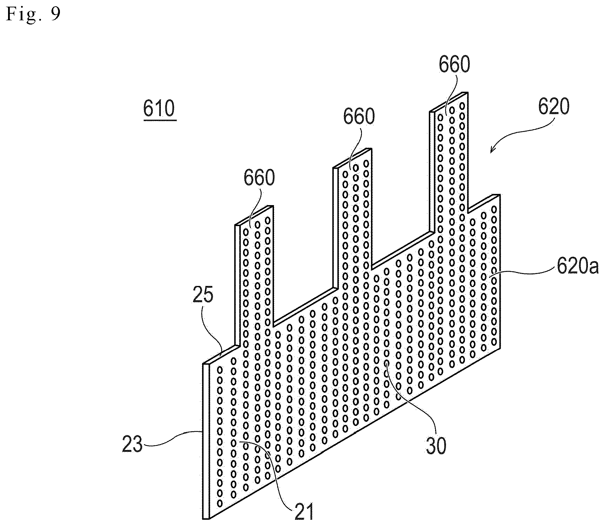

Fig. 9 illustrates an overview perspective view of a medical apparatus 610 (a biodegradable sheet 620) according to Modification Example 6.

As illustrated in Fig. 9, the biodegradable sheet 620 according to Modification Example 6 includes a main body portion 620a having an approximately rectangular shape in a plan view. In addition, the biodegradable sheet 620 includes a protruding portion 660 protruding from one side (the lateral surface 25) of the main body portion 620a.

A plurality of protruding portions 660, for example, can be provided on one lateral surface 25 of the main body portion 620a. In addition, an arbitrary number of protruding portions 620, for example, can be provided on each of a plurality of lateral surfaces 25 of the main body portion 620a. In this modification example, three protruding portions 660 are provided on one lateral surface 25 of the main body portion 620a. Note that, the shape of the protruding portion 660, the length of the protruding direction, a width in a direction intersecting with the protruding direction, a pitch between the protruding portions 660, and the like, are not particularly limited.

As with the medical apparatus 610 according to this modification example, in a case where the biodegradable sheet 620 has a rectangular shape in a plan view, and includes the protruding portion 660 protruding from one side of the biodegradable sheet 620, in a predetermined manipulation (triangular anastomosis of the esophagus), it is possible to preferably use the biodegradable sheet 620. In a case of using the biodegradable sheet 620 in the manipulation described above, for example, a support string is applied to the end portions of the esophagus (the end portions of the esophagus in a cutoff state) that is an anastomosis target and is lifted, and thus, the protruding portion 660 is allowed to pass through a gap between the support strings. Then, the end portions of the esophagus, facing each other through the main body portion 620a of the biodegradable sheet 620, are sutured. Hereinafter, similarly, an operation of lifting the support string and of allowing the protruding portion 660 to pass through the gap between the support strings is performed in a different position in a circumferential direction of the end portion of the esophagus. By performing the operation described above, the end portions of the esophagus are sutured in three sites in the circumferential direction of the end portion of the esophagus.

### <Modification Example 7>

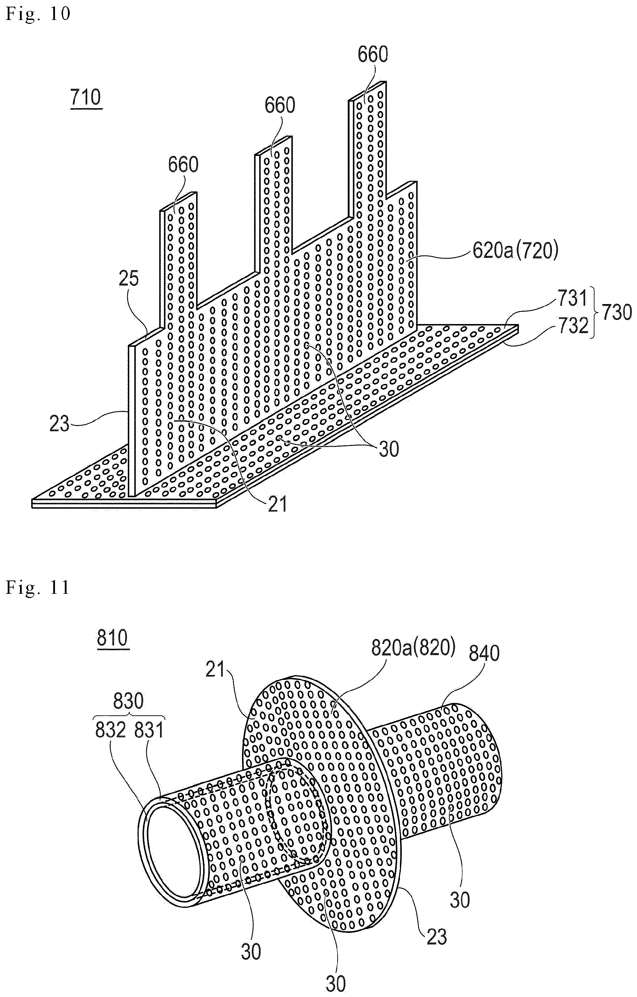

Fig. 10 illustrates an overview perspective view of a medical apparatus 710 according to Modification Example 7.

As illustrated in Fig. 10, the medical apparatus 710 according to Modification Example 7 includes the main body portion 620a including the biodegradation sheet 720, the protruding portion 660, and a guide portion 730. The main body portion 620a and the protruding portion 660 can be configured substantially the same as those of the medical apparatus 610 according to Modification Example 6 described above.

The guide portion 730 includes a first sheet member 731 in which the through-holes 30 are formed, and a second sheet member 732 that is disposed to be stacked on the first sheet member 731. The through-holes 30 are not formed in the second sheet member 732.

The second sheet member 732, for example, can be configured to have rigidity (have hard physical properties) higher than that of the first sheet member 731. As an example, the first sheet member 731 and the second sheet member 732 contain the same material as that of the main body portion 620a, and the through-holes 30 are formed in the first sheet member 731, and thus, it is possible to adjust a magnitude relationship between the rigidity of the first sheet member 731 and the rigidity of the second sheet member 732.

The medical apparatus 710 according to this modification example, for example, can be preferably used in triangular anastomosis of the esophagus. The guide portion 730 of the medical apparatus 710 is disposed on the outer circumference side of the esophagus that is an anastomosis target, and thus, is attached to the outer wall of the end portion of the esophagus. The guide portion 730 is in contact with the outer wall of the end portion of the esophagus, and thus, it is possible to position the main body portion 620a on the lumen side of the esophagus. In addition, when the end portions of the esophagus are sutured, and a surplus portion of the medical apparatus 710 is ablated along with a part of the esophagus, the guide portion 730 disposed on the outer wall side of the end portion of the esophagus functions as a guide for designating an ablation position. For this reason, it is possible to smoothly perform an ablation operation.

The guide portion 730, for example, may have a structure not including the first sheet member 731. The guide portion 730 includes at least the second sheet member 732 having rigidity higher than that of the first sheet member 731, and thus, it is possible to preferably exhibit a function of guiding the ablation position. However, in a case where the guide portion 730 includes the first sheet member 731, it is possible to more preferably promote the adhesion of the esophagus by disposing the first sheet member 731 to be in contact with the outer circumference side of the end portion of the esophagus that is an anastomosis target.

### <Modification Example 8>

Fig. 11 illustrates an overview perspective view of a medical apparatus 810 according to Modification Example 8.

As illustrated in Fig. 11, the medical apparatus 810 according to Modification Example 8 includes a main body portion 820a including a biodegradation sheet 820, a first hollow portion 830 protruding to one end side of the main body portion 820a, and a second hollow portion 840 protruding to the other end side of the main body portion 820a.

The first portion 830 includes an approximately cylindrical outer member 831 in which the through-holes 30 are formed, and an approximately cylindrical inner member 832 disposed on the inner surface of the outer member 831.

The outer member 831 and the inner member 832 contain the same material as that of the biodegradable sheet 820. However, the through-holes 30 are not formed in the inner member 832. A flow path in which body fluid (pancreatic juice or the like) can be circulated is formed in the lumen of the inner member 832.

The second portion 840 is formed substantially the same as the outer member 831. The second portion 840 contains the same material as that of the biodegradable sheet 820, and has the through-holes 30.

The medical apparatus 810 according to this modification example, for example, can be preferably used in pancreatic parenchyma-jejunum seromuscular anastomosis. The first portion 830 of the medical apparatus 810, for example, can be inserted into an opening portion formed in the jejunum. The outer member 831 of the first portion 830 is disposed by being attached to the jejunum, and thus, the adhesion with respect to the jejunum is promoted. On the other hand, the through-holes 30 are not formed in the inner member 832 of the first portion 830, and thus, it is possible to prevent collapse or kink. For this reason, it is possible to maintain the flow path formed by the lumen of the inner member 832 in a suitable shape, and it is possible to preferably prevent the occurrence of stenosis due to the narrowing of the lumen of the inner member 832. The main body portion 820a can be disposed to be interposed between the jejunum and the pancreatic parenchyma. In addition, the second portion 840 can be inserted into the ductus pancreaticus. The through-holes 30 are formed in the main body portion 820a and the second portion 840, and thus, it is possible to preferably promote the adhesion in the jejunum, the pancreatic parenchyma, and the ductus pancreaticus.

The configurations described in each of the modification examples described above can be suitably combined. For example, the communicating holes 31 formed in the lateral surface 25 described in Modification Example 2 can also be formed in the biodegradable sheets 410, 510, and 610 described in Modification Examples 5, 6, and 7. In addition, for example, through-holes including the narrow portion 230a described in Modification Example 3 and the wide portion 330a described in Modification Example 4 can also be formed in one biodegradable sheet, and the narrow portion 230a and the wide portion 330a can also be provided in one through-hole.

### Examples

The effects of the invention will be described by using the following examples and comparative examples. However, the technical scope of the invention is not limited only to the following examples.

### <Manufacturing Example of Biodegradable Sheet (Adhesion Promoting Device)>

A biodegradable sheet (an adhesion promoting device) in which a plurality of through-holes were formed, as illustrated in Fig. 1 and Fig. 2, was manufactured by irradiating a biodegradable resin sheet formed by an electrospinning method with laser light. Here, the values of the hole diameter (D), the pitch (P), the Hole Diameter/Pitch ratio (D/P), and the thickness (T) of the plurality of through-holes formed in the obtained biodegradable sheet (adhesion promoting device) are shown in Table 1 described below.

An adhesion promoting effect of the biodegradable sheet (the adhesion promoting device) manufactured as described above was evaluated by using a rabbit abdominal wall cecum synechia model.

Specifically, a female Japanese white rabbit) (3 ± 0.5 kg) was prepared as a model animal. As a surgical procedure, the peritoneum was subjected to midline section along a white line on the abdominal wall to have a length of 10 cm, and then, a deficit of 3 cm × 4 cm, including the parietal peritoneum and the muscular layer, was prepared on both right and left abdominal walls from a position separated from a midline by 1 cm. Then, four corners of a size of 2 cm × 3 cm were sutured by a string, and thus, the abdominal wall and the cecum were fixed.

The biodegradable sheet (the adhesion promoting device) manufactured as described above was interposed between the abdominal wall and the cecum in a fixing portion by using such an abdominal wall cecum synechia model, without imparting a damage to the fixing portion. Then, autopsy was performed after 3 days (72 hours) during which the brittleness of a biological tissue after a surgery reached a peak, and the degree of synechia at this time was evaluated in accordance with the following grades. The results are shown in Table 1 described below. Note that, in this example, the value of the grade in each test example is an arithmetic average value of values obtained by performing the same test with respect to each of model animals of the numbers (total 18 heads) shown in Table 1 described below. In addition, in the abdominal wall cecum synechia model, in a non-procedure group subjected to the same test without using the biodegradable sheet, the degree of synechia after 3 days (72 hours) was Grade 0. In addition, in a damaged group subjected to the same test after imparting a damage to the fixing portion instead of using the biodegradable sheet, the degree of synechia after 3 days (72 hours) was Grade 3.

Grade 0: No adhesion is observed (peeled off under its own weight)
Grade 1: Adhesion that can be peeled off by a slight blunt manipulation without any tissue damage
Grade 2: Adhesion that can be peeled off by a blunt manipulation without any tissue damage
Grade 3: Adhesion that can be peeled off by a blunt manipulation with a tissue damage
Grade 4: Adhesion that can be peeled off by a strong blunt manipulation with a tissue damage
Grade 5: Adhesion that is not capable of being peeled off even by a strong blunt manipulation

**[Table 1]**

| | Hole diameter D (mm) | Pitch P (mm) | Hole diameter/pitch ratio D/P | Thickness T (mm) | Synechia grade | Numbers |
|---|---|---|---|---|---|---|
| Example 1 | 0.3 | 0.1 | 3 | 0.1 | 3 | 2 |
| Example 2 | 0.6 | 0.2 | 3 | 0.1 | 3 to 3.5 | 5 |
| Example 3 | 0.6 | 0.2 | 3 | 0.2 | 4 to 5 | 3 |
| Example 4 | 0.6 | 0.4 | 1.5 | 0.1 | 3.5 to 4 | 3 |
| Example 5 | 0.6 | 0.4 | 1.5 | 0.2 | 4 | 1 |
| Example 6 | 1.0 | 0.2 | 5 | 0.2 | 4 | 1 |
| Example 7 | 4.0 | 0.2 | 20 | 0.2 | 4 | 2 |
| Comparative Example 1 | 8.0 | 0.2 | 40 | 0.2 | 2 | 1 |

From the results shown in Table 1, it is found that an excellent adhesion promoting effect can be obtained by the adhesion promoting device using the medical apparatus (the biodegradable sheet) according to the invention in which the value of the Hole Diameter/Pitch ratio (D/P) is 1.5 or more and 20 or less.

### <Manufacturing Example of Biodegradable Sheet (Adhesion Promoting Device) Containing Adhesion Enhancement Agent>

A biodegradable sheet (an adhesion promoting device) in which a plurality of through-holes were formed, as illustrated in Fig. 1 and Fig. 2, was manufactured by using two types of formulations. In the first formulation, the biodegradable sheet (an adhesion promoting device) was manufactured by a method in which a biodegradable resin sheet formed by an electrospinning method in a state where an adhesion enhancement agent was dissolved or suspended in a resin solution was subjected to press hole processing (hereinafter, also referred to as a "kneading method"). In the second formulation, the biodegradable sheet (an adhesion promoting device) was manufactured by a method in which a biodegradable resin sheet formed by an electrospinning method only with a resin solution was subjected to press hole processing, and then, was immersed in a solution of an adhesion enhancement agent, and was dried, and thus, the adhesion enhancement agent was adsorbed on the biodegradable sheet (hereinafter, also referred to as an "adsorption method").

Here, the hole diameters (D) of the plurality of through-holes provided in the biodegradable sheet (the adhesion promoting device) containing the obtained adhesion enhancement agent were unified at 0.6 mm, the pitches (P) were unified at 0.2 mm, and the thicknesses (T) were unified at 0.1 mm. The type of adhesion enhancement agent, a blending ratio, and a manufacturing method are shown in Table 2 described below. Note that, the value of the blending ratio of the adhesion enhancement agent represents a blending amount of the adhesion enhancement agent by mass% when the mass of the biodegradable sheet is 100 mass%.

An adhesion promoting effect of the biodegradable sheet (the adhesion promoting device) containing the adhesion enhancement agent manufactured as described above was evaluated by using the rabbit abdominal wall cecum synechia model described above.

**[Table 2]**

| | Adhesion enhancement agent | Blending ratio (%) | Manufacturing method | Synechia grade | Numbers |
|---|---|---|---|---|---|
| Example 8 | Talc | 50 | Kneading method | 5 | 2 |
| Example 9 | Povidone-iodine | 50 | | 4.5 | 1 |
| Example 10 | | 73 | Adsorption method | 5 | 1 |
| Example 11 | Talc/povidone-iodine | 67/8 | | 5< | 1 |
| Example 12 | Minocycline | 21 | | 4.5 | 1 |
| Comparative Example 2 | - | - | - | 4 | 2 |

From the results shown in Table 2, it is found that an excellent adhesion promoting effect is obtained by the adhesion promoting device using the medical apparatus (the biodegradable sheet) containing the adhesion enhancement agent.

As described above, the invention provides means for reducing the risk of a ruptured suture after a surgery or the like by a simple technique, and is extremely clinically useful.

### Reference Signs List

[0085]
10, 110, 210, 310, 410, 510, 610, 710, 810 Medical apparatus
20, 120, 220, 320, 420, 520, 620, 720, 820 Biodegradable sheet
21 One surface
23 Other surface
25 Lateral surface (one side)
30 Through-hole
31 Communicating hole
230a Narrow portion
330a Wide portion
440 Central hole
550 Slit
620a Main body portion of biodegradable sheet
660 Protruding portion
D Hole diameter of through-hole
P Pitch of through-hole
T Thickness of biodegradable sheet

## Claims

1. An adhesion promoting device (10), comprising:
a biodegradable sheet (20) in which a plurality of through-holes (30) are formed,
wherein when the adhesion promoting device (10) is applied to anastomosis sites, a biological reaction is caused to induce the expression of a biological component, and the biological component induced is accumulated through the through-holes (30), thereby promoting adhesion in the anastomosis sites,
wherein the biodegradable sheet (20) comprises a fiber containing a biodegradable resin and is in the shape of a mesh, and a value of a ratio (D/P) of a hole diameter D to a pitch P of the through-holes (30) is 1.5 to 20,
wherein the hole diameter D is 0.1 mm to 6 mm,
the pitch P is 0.1 mm to 0.4 mm, and
a thickness of the biodegradable sheet (20) is 0.1 mm to 0.2 mm.

2. The adhesion promoting device (10) according to claim 1,
wherein the through-holes (30) are provided approximately perpendicularly along a thickness direction of the biodegradable sheet (20).

3. The adhesion promoting device (10) according to claim 1 or 2,
wherein the through-holes (30) have a shape in which a part widens in a direction intersecting with the thickness direction of the biodegradable sheet (20).

4. The adhesion promoting device (10) according to any one of claims 1 to 3,
wherein the biodegradable sheet (20) has a circular shape in a plan view, and
a central hole (440) having a hole diameter larger than that of the through-holes (30) is formed in an approximately central position in the plan view.

5. The adhesion promoting device (10) according to claim 4, further comprising:
a slit (550) continuing to a lateral surface of the biodegradable sheet (20) and the central hole (440).

6. The adhesion promoting device (10) according to any one of claims 1 to 3,
wherein the biodegradable sheet (20) includes a main body portion (620a) formed in an approximately rectangular shape in a plan view, and
a projection portion (660) protruding from the main body portion (620a) is formed on one side of the main body portion (620a).

7. The adhesion promoting device (10) according to any one of claims 1 to 6,
wherein a constituent material of the biodegradable sheet (20) is a polylactic acid (PLA), a polyglycolic acid (PGA), or a lactic acid-glycolic acid copolymer (PLGA).

8. The adhesion promoting device (10) according to any one of claims 1 to 7,
wherein an adhesion enhancement agent is blended in the biodegradable sheet (20).

## Patentansprüche

1. Haftungsfördernde Vorrichtung (10), die Folgendes umfasst:
eine biologisch abbaubare Folie (20), in der eine Vielzahl von Durchgangslöchern (30) ausgebildet sind,
wobei dann, wenn die haftungsfördernde Vorrichtung (10) auf Anastomosestellen aufgebracht wird, eine biologische Reaktion hervorgerufen wird, um die Expression einer biologischen Komponente herbeizuführen, und wobei die herbeigeführte biologische Komponente durch die Durchgangslöcher (30) hindurch angesammelt wird, wodurch die Haftung an den Anastomosestellen gefördert wird,
wobei die biologische abbaubare Folie (20) eine Faser umfasst, die ein biologisch abbaubares Harz enthält und in Form eines Netzes vorliegt, und wobei ein Wert eines Verhältnisses (D/P) eines Lochdurchmessers D zu einem Abstand P der Durchgangslöcher (30) 1,5 bis 20 beträgt,
wobei der Lochdurchmesser D 0,1 mm bis 6 mm beträgt,
der Abstand P 0,1 mm bis 0,4 mm beträgt, und
eine Dicke der biologisch abbaubaren Folie (20) 0,1 mm bis 0,2 mm beträgt.

2. Haftungsfördernde Vorrichtung (10) nach Anspruch 1,
wobei die Durchgangslöcher (30) ungefähr senkrecht entlang einer Dickenrichtung der biologisch abbaubaren Folie (20) vorgesehen sind.

3. Haftungsfördernde Vorrichtung (10) nach Anspruch 1 oder 2,
wobei die Durchgangslöcher (30) eine Form haben, bei der ein Teil in einer Richtung, die sich mit der Dickenrichtung der biologisch abbaubaren Folie (20) schneidet, breiter wird.

4. Haftungsfördernde Vorrichtung (10) nach einem der Ansprüche 1 bis 3,
wobei die biologisch abbaubare Folie (20) in einer Draufsicht eine kreisrunde Form hat, und
ein mittiges Loch (440) mit einem Lochdurchmesser, der größer ist als der der Durchgangslöcher (30), in der Draufsicht in einer ungefähr mittigen Position ausgebildet ist.

5. Haftungsfördernde Vorrichtung (10) nach Anspruch 4, die ferner Folgendes umfasst:
einen Schlitz (550), der bis zu einer Seitenfläche der biologisch abbaubaren Folie (20) und dem mittigen Loch (440) verläuft.

6. Haftungsfördernde Vorrichtung (10) nach einem der Ansprüche 1 bis 3,
wobei die biologisch abbaubare Folie (20) einen Hauptkörperabschnitt (620a) aufweist, der in einer Draufsicht in einer ungefähr rechteckigen Form ausgebildet ist, und
ein Vorsprungsabschnitt (660), der von dem Hauptkörperabschnitt (620a) vorspringt, auf einer Seite des Hauptkörperabschnitts (620a) ausgebildet ist.

7. Haftungsfördernde Vorrichtung (10) nach einem der Ansprüche 1 bis 6,
wobei ein konstituierendes Material der biologisch abbaubaren Folie (20) eine Polymilchsäure (PLA), eine Polyglycolsäure (PGA) oder ein Milchsäure-Glycolsäure-Copolymer (PLGA) ist.

8. Haftungsfördernde Vorrichtung (10) nach einem der Ansprüche 1 bis 7,
wobei ein Haftvermittler in die biologisch abbaubare Folie (20) eingearbeitet ist.

## Revendications

1. Dispositif favorisant l'adhérence (10), comprenant :
une feuille biodégradable (20) dans laquelle une pluralité de trous traversants (30) sont formés,
dans lequel, lorsque le dispositif favorisant l'adhérence (10) est appliqué sur des sites d'anastomose, une réaction biologique est provoquée pour induire l'expression d'une composante biologique, et la composante biologique induite est accumulée à travers les trous traversants (30), favorisant ainsi l'adhérence dans les sites d'anastomose, dans lequel la feuille biodégradable (20) comprend une fibre contenant une résine biodégradable et a la forme d'un maillage, et une valeur d'un rapport (D/P) d'un diamètre de trou D à un entraxe P des trous traversants (30) est de 1,5 à 20,
dans lequel le diamètre de trou D est de 0,1 mm à 6 mm, l'entraxe P est de 0,1 mm à 0,4 mm, et
une épaisseur de la feuille biodégradable (20) est de 0,1 mm à 0,2 mm.

2. Dispositif favorisant l'adhérence (10) selon la revendication 1,
dans lequel les trous traversants (30) sont prévus approximativement perpendiculairement le long d'une direction d'épaisseur de la feuille biodégradable (20).

3. Dispositif favorisant l'adhérence (10) selon la revendication 1 ou 2,
dans lequel les trous traversants (30) ont une forme dans laquelle une partie s'élargit dans une direction croisant la direction d'épaisseur de la feuille biodégradable (20).

4. Dispositif favorisant l'adhérence (10) selon l'une quelconque des revendications 1 à 3,
dans lequel la feuille biodégradable (20) a une forme circulaire dans une vue en plan, et
un trou central (440) ayant un diamètre de trou plus grand que celui des trous traversants (30) est formé à une position approximativement centrale dans la vue en plan.

5. Dispositif favorisant l'adhérence (10) selon la revendication 4, comprenant en outre :
une fente (550) se poursuivant jusqu'à une surface latérale de la feuille biodégradable (20) et le trou central (440).

6. Dispositif favorisant l'adhérence (10) selon l'une quelconque des revendications 1 à 3,
dans lequel la feuille biodégradable (20) comporte une portion corps principal (620a) ayant une forme approximativement rectangulaire dans une vue en plan, et une portion saillie (660) faisant saillie à partir de la portion corps principal (620a) est formée sur un côté de la portion corps principal (620a).

7. Dispositif favorisant l'adhérence (10) selon l'une quelconque des revendications 1 à 6,
dans lequel un matériau constitutif de la feuille biodégradable (20) est un acide polylactique (PLA), un acide polyglycolique (PGA) ou un copolymère d'acide lactique-acide glycolique (PLGA).

8. Dispositif favorisant l'adhérence (10) selon l'une quelconque des revendications 1 à 7,
dans lequel un agent d'amélioration d'adhérence est mélangé dans la feuille biodégradable (20).
